(19) Europäisches Patentamt
European Patent Office
Office européen des brevets

(11) EP 0 702 667 B1

(12) **FASCICULE DE BREVET EUROPEEN**

(45) Date de publication et mention de la délivrance du brevet:
**21.01.1998 Bulletin 1998/04**

(21) Numéro de dépôt: **94918893.2**

(22) Date de dépôt: **08.06.1994**

(51) Int Cl.[6]: **C07C 29/147**, C07C 31/20, C07C 29/80

(86) Numéro de dépôt international:
**PCT/FR94/00673**

(87) Numéro de publication internationale:
**WO 94/29253 (22.12.1994 Gazette 1994/28)**

(54) **PROCEDE DE PREPARATION DE COMPOSES DIFONCTIONNELS DE HAUTE PURETE ENANTIOMERIQUE**

VERFAHREN ZUR HERSTELLUNG VON HOCH ENANTIOMERENREINEN DIFUNKTIONELLEN VERBINDUNGEN

METHOD FOR PREPARING DIFUNCTIONAL COMPOUNDS HAVING HIGH ENANTIOMERIC PURITY

(84) Etats contractants désignés:
**AT BE CH DE DK ES FR GB GR IE IT LI LU MC NL PT SE**

(30) Priorité: **11.06.1993 FR 9307280**

(43) Date de publication de la demande:
**27.03.1996 Bulletin 1996/13**

(73) Titulaire: **RHONE-POULENC CHIMIE**
**92408 Courbevoie Cédex (FR)**

(72) Inventeur: **RADISSON, Xavier**
**F-69003 Lyon (FR)**

(74) Mandataire: **Ricalens, François**
**RHONE-POULENC CHIMIE,**
**Direction de la Propriéte Industrielle,**
**25, quai Paul Doumer**
**92408 Courbevoie Cédex (FR)**

(56) Documents cités:
**EP-A- 0 350 852**

- **CHEMISCHE BERICHTE, vol.109, 1976, WEINHEIM DE pages 2645 - 2647 J GOMBOS ET AL 'NOTIZ ÜBER EINE EINFACHE HERSTELLUNG VON (S)-PROPYLENOXID'**
- **CHEMISTRY AND INDUSTRY. CHEMISTRY AND INDUSTRY REVIEW, vol.6, 1976, LETCHWORTH GB page 218 C MELCHIORRE 'A CONVENIENT SYNTHESIS OF S(+)-PROPANE-1,2-DIOL' cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 702 667 B1

Printed by Jouve, 75001 PARIS (FR)

## Description

Le domaine de l'invention est celui des énantiomères, en particulier difonctionnels, de haute pureté optique.

Cette pureté est particulièrement intéressante et avantageuse au regard de l'expression optimale des fonctionnalités des énantiomères, notamment en tant que réactifs, synthons ou intermédiaires dans des synthèses spécifiques de composés chiraux, tels que des principes actifs, e. g. pharmaceutiques ou agro-chimiques, ou en tant que composés actifs en eux-mêmes ou bien encore comme précurseurs de polymères spéciaux.

Plus précisément, la présente invention concerne la synthèse de composés difonctionnels de haute pureté énantiomérique et de formule :

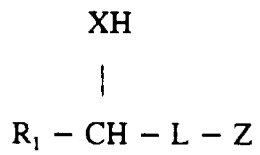

**(If)**

dans laquelle :

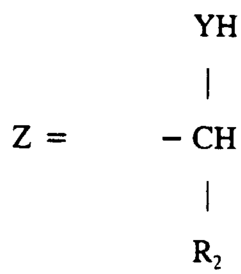

| | |
|---|---|
| $R_1$, $R_2$ | sont identiques ou différents et représentent l'hydrogène ou un radical hydrocarboné aliphatique et/ou alicyclique et/ou aromatique et/ou hétérocyclique, de préférence un hydrogène ou un alkyle, |
| X et Y | sont identiques ou différents et représentent l'oxygène ou le soufre, |
| et L | représente une simple liaison covalente a ou un groupement alkyle à 1 ou 2 C, |

**à partir** de composés chiraux purs répondant à la formule **(Id)**, qui correspond à la formule **(If)** dans laquelle :

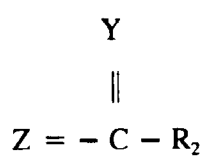

avec $R_2$ et Y tels que définis ci-dessus, $R_2$ pouvant, en outre, représenter un radical $NH_2$, alcoxyle, S-alkylé ou :

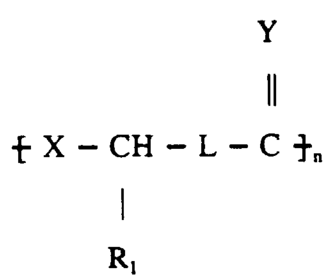

avec n = 1 à 1 000.

Plus précisément encore, la présente invention est relative à la synthèse d'énantiomères de haute pureté optique comprenant au moins deux fonctions hydroxyles (diols) ou deux fonctions thiols (dithiols).

Un exemple non limitatif de diol est le (+) (S) 1,2 propane-diol ou (-) (R) 1,2 propane-diol.

L'obtention d'énantiomères de haute pureté optique s'opère, traditionnellement, par résolution du racémique : voie chimique ou enzymatique. La voie chimique est, essentiellement, celle de la conversion du racémique en deux diastéréoisomères séparables. On peut citer la séparation par chromatographie phase liquide ou par cristallisation.

Ces résolutions chimiques sont lourdes, complexes, donc onéreuses à mettre en oeuvre.

Il en va de même pour les résolutions enzymatiques qui présentent, en outre, l'inconvénient d'être spécifiques d'un énantiomère donné.

On connaît, également, des voies de synthèse chimique asymétrique spécifique comme, par exemple, la dihydroxylation asymétrique du propène, catalysée par le tétraoxyde d'osmium et permettant d'obtenir l'un des énantiomères du propane 1,2 diol (K. B. SHARPLESS et al., J. o. c., 1992, 57, 2768), avec une pureté énantiomérique qui reste modeste.

Une autre possibilité, plus avantageuse, d'obtention de composés de haute pureté énantiomérique, industriellement exploitables, consiste à utiliser, en tant que produits de départ, des composés chiraux, comme par exemple ceux répondant à la formule **(Id)** donnée ci-dessus et à les réduire de manière à produire les dérivés hydrogénés de haute pureté énantiomérique visés **(If)**.

Par définition, les produits chiraux **(Id)** sont peu coûteux, car aisément disponibles ou accessibles. Il peut s'agir de produits présents dans la nature et aisément extractibles, ou bien encore de composés pouvant être produits en quantités industrielles, e. g. par fermentation ou dédoublement. Ces produits **(Id)** sont, par exemple, des esters d'acides, $\alpha$, $\beta$ ou $\gamma$ hydroxycarboxyliques ou d'autres dérivés de ceux-ci, tels que des cétones ou des aldéhydes, ainsi que leurs équivalents, lorsque l'on substitue le soufre à l'oxygène dans les radicaux X, Y.

Dans ces techniques mettant en oeuvre une réduction, on retiendra celles par réduction catalytique sous hydrogène, ainsi que celles par réduction stoechiométrique à l'aide d'un agent réducteur chimique.

En ce qui concerne la réduction catalytique sous hydrogène, la littérature technique antérieure témoigne des grandes difficultés qui existent pour réduire un acide, un ester, un aldéhyde, une cétone ou leurs analogues soufrés, en alcool ou thiol dans des conditions douces et productives. Les catalyseurs utilisés, en catalyse hétérogène, généralement sont le nickel de Raney et les chromites de cuivre.

A titre d'exemple, on peut citer l'article de E. BOWDEN et COLL, JACS, 56, 689, 1934, qui est l'un des seuls à se préoccuper de la chiralité et qui signale une racémisation totale, lorsque l'on soumet du lactate de N-butyle (+) à une réduction par catalyse hétérogène, à l'aide de chromite de cuivre (200 bars - 225° C - 2 heures en masse).

Il faut donc retenir que les conditions réactionnelles imposées par la réduction catalytique sont trop drastiques et entraînent, de fait, une racémisation ou une perte de chiralité des produits finaux obtenus par réduction d'acides hydroxycarboxyliques. de leurs esters, d'hydroxycétones ou d'hydroxyaldéhydes.

Accessoirement, il convient de signaler, également, la faible productivité de ces techniques de réduction catalytique.

La réduction stoechiométrique par réducteur chimique se résume, essentiellement, à l'utilisation d'hydrures en tant que réducteurs.

Les hydrures principalement concernés sont le borohydrure de lithium ($LiBH_4$), le borohydrure de sodium ($NaBH_4$), le borohydrure de potassium ($KBH_4$), l'hydrure d'aluminium lithium ($LiAlH_4$) ou l'hydrure de diisobutyle aluminium (DiBAH).

Il existe de nombreuses publications scientifiques traitant de la réduction à l'aide d'hydrures, d'énantiomères purs d'acides alpha-hydroxycarboxyliques ou de leurs esters (acide lactique/lactate) en énantiomères plus ou moins purs, du type diol : propane-1,2 diol.

A titre d'exemple, on peut citer l'article de BARNETT et KENT, JCS, 2743-510 (1963), décrivant la transformation de pyruvate de méthyle, en milieu alcoolique à 20° C et en présence de borohydrure de potassium, en un mélange de lactate de méthyle et de propane-1,2 diol. Dans cette réaction, le métal complexé avec l'hydrogène dans l'hydrure (bore) se lie avec le substrat, donc avec le produit final. Cela impose de déplacer le complexe métallique ainsi formé (borate) par un traitement consistant, tout d'abord, à procéder à une dilution avec du méthanol et à acidifier avec une solution méthanolique acide d'HCl. Il se forme ainsi du borate de méthyle, qui est distillé à pression atmosphérique. Il est ensuite nécessaire de neutraliser la solution à l'aide de carbonate de plomb, de filtrer et de concentrer sous pression réduite le produit, qui est finalement extrait par distillation fractionnée ou par entraînement au chloroforme. Selon une autre variante, on extrait le produit final par entraînement avec un système solvant éther-eau et distillation fractionnée.

Une telle technique ne permet pas d'atteindre de bons rendements et nécessite, par ailleurs, des traitements de purification et d'extraction longs, lourds et complexes à mettre en oeuvre.

MELCHIORRE décrit, dans "Chemistry and Industry, 6 march 1976, page 218", une synthèse de S (+) propane 1,2 diol à partir de S (+) acide lactique et en présence de diborane. Le milieu solvant employé est du tétrahydrofurane. L'élimination de l'excès de diborane s'opère par l'addition d'eau et le déplacement du complexe bore/propanediol par

rinçage au méthanol anhydre, ce solvant induisant une transestérification et une formation de borate triméthylé. Le solvant est ensuite évaporé. Cette opération de dilution-évaporation est répétée jusqu'à élimination complète du bore.

Outre le fait que le diborane est un réactif dangereux à manipuler, le fait de faire intervenir de l'eau limite nécessairement les rendements, car les esters de bore et de propane-1,2 diol sont extrêmement solubles dans ce solvant, d'où des difficultés certaines d'extraction. Le rendement de MELCHIORRE n'est, en effet, que de 75 %, tandis que la pureté optique de l'énantiomère obtenu est de $[\alpha]^{25}_D = +15,84°$, soit un excès énantiomérique :

$$ee = \frac{\text{proportion d'énantiomère majoritaire - proportion d'énantiomère minoritaire}}{\text{proportion d'énantiomère majoritaire + proportion d'énantiomère minoritaire}} = 92,$$

ce qui reste relativement faible.

Enfin, il faut indiquer que la procédure de dissolution méthanolique/évaporation n'est pas vraiment industrielle.

Pour en terminer avec l'illustration de l'état de la technique, il convient de mentionner le brevet US n° 4 945 187 décrivant un procédé de préparation d'énantiomères purs de S (+) propane 1-2 diol et de R (-) propane 1-2 diol à partir de L (-) lactide et de D (+) lactide, respectivement. La réduction s'opère ici dans le toluène à l'aide d'hydrure de diisobutyle aluminium (DiBAH). Le déplacement du complexe avec les restes d'hydrure est effectué par hydrolyse à l'aide de NaOH, puis par précipitation de l'hydroxyde d'aluminium, en solution aqueuse à pH acide. On récupère ensuite les énantiomères par évaporation. lavage à l'éthanol, puis distillation.

L'eau, avec tout ce qu'elle représente de pénalisant, est encore employée dans ce procédé qui, en outre, comporte de multiples étapes longues et complexes à mettre en oeuvre et donc, in fine, difficiles à mettre en oeuvre industriellement.

Il ressort clairement de ce qui précède qu'il existe une carence en un procédé de préparation d'énantiomères de haute pureté optique, c'est-à-dire ayant un excès énantiomérique ee supérieur à 95 %, de composés du type diol ou dithiol ou bien encore mono OH et mono SH, à partir de dérivés d'acides $\alpha$, $\beta$ ou $\gamma$, de préférence a, hydroxycarboxyliques, tels que les esters, les dimères cycliques, les cétones, ou les aldéhydes,
qui soit :

◁ économique,
◁ aisé à mettre en oeuvre,
◁ rapide,

et qui réponde aux contraintes industrielles de haut rendement et grande productivité.

L'un des objets de la présente invention est donc de remédier à cette carence en proposant un procédé de préparation de composés difonctionnels de haute pureté énantiomérique, du type de ceux énoncés ci-dessus et, en particulier mais non limitativement, de diol-1,2 chiraux, à partir des dérivés des $\alpha$-hydroxy acides correspondants, ledit procédé devant permettre la réduction douce du substrat chiral, de manière à éviter la racémisation et devant offrir les conditions optimales d'extraction rapide et aisée de l'énantiomère pur visé, notamment en évitant le recours à l'eau, qui perturbe inévitablement l'extraction.

Aussi, c'est après de longues et laborieuses recherches et expériences que la Demanderesse a mis en évidence, de façon tout à fait surprenante et inattendue, que, dans la synthèse de composés difonctionnels de haute pureté énantiomérique par réduction stoechiométrique à l'aide d'hydrures :

◁ le milieu réactionnel est avantageusement anhydre (solvant organique),
◁ la phase de déplacement du composé difonctionnel visé, du complexe qu'il forme avec le produit de transformation de l'hydrure (borate) est, avantageusement, effectuée à l'aide d'au moins un coréactif:

  ◁◁ substitué par au moins un radical hydroxyle ou thiol, de préférence au moins deux,
  ◁◁ et possédant une température d'ébullition à la pression atmosphérique normale ($P_{atm}$) supérieure à celle du composé bifonctionnel visé,

◁ et qu'il est également tout à fait judicieux de procéder à une distillation directe, permettant de séparer l'énantiomère visé (di OH, di SH ou mono OH et mono SH).

Ainsi, la présente invention concerne un procédé de préparation de composés difonctionnels, de haute pureté énantiomérique et de formule :

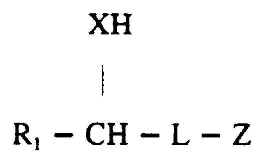

**(If)**

dans laquelle :

- 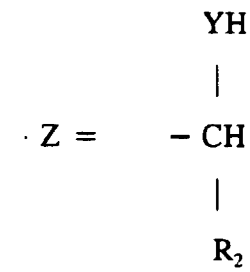
- $R_1$, $R_2$ sont identiques ou différents et représentent l'hydrogène ou un radical et/ou hydrocarboné aliphatique et/ou alicyclique et/ou aromatique et/ou hétérocyclique, de préférence un hydrogène ou un alkyle,
- X et Y sont identiques ou différents et représentent l'oxygène ou le soufre,
- et L représente une simple liaison covalente a ou un groupement alkyle à 1 ou 2 C,

à partir de composés chiraux purs répondant à la formule (**Id**) qui correspond à la formule (**If**) dans laquelle :

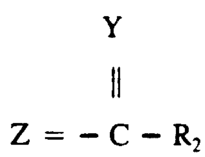

avec $R_2$ et Y tels que définis ci-dessus, $R_2$ pouvant, en outre, représenter un radical

- $NH_2$, alcoxyle, S alkylé ou :

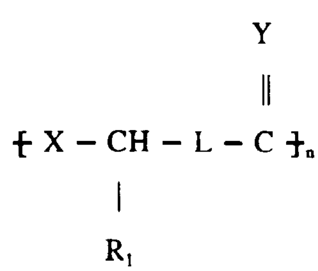

avec n = 1 à 1 000, de préférence 1 à 500 et, plus préférentiellement encore, de 1 à 100,
procédé du type de ceux faisant intervenir une réduction du composé (**Id**) à l'aide d'au moins un réducteur du type hydrure,

caractérisé en ce que, en milieu anhydre :

**a)** - on effectue la réduction du composé **(Id)** en composé **(If)** dans un milieu solvant organique,
**b)** - on ajoute au milieu réactionnel au moins un coréactif:

- substitué par au moins un radical XH ou YH, de préférence au moins deux,
- apte à déplacer le composé **(If)** dans l'intermédiaire issu de l'étape a) précédente,
- et possédant une température d'ébullition à pression atmosphérique normale ($P_{atm}$) supérieure à celle du

composé **(If)**,

**c)** - on distille directement le milieu réactionnel de manière à récupérer le composé **(If)**.

Conformément à l'invention, les produits de départ de formule **(Id)** visés peuvent être des dérivés (esters, dimères cycliques ou non, oligo ou polycondensats, aldéhydes, cétones) d'acides carboxyliques α, β ou γ hydroxylés, de préférence α, ainsi que les homologues soufrés de ces composés oxygénés.

Quant le substrat de départ est un dimère cyclique ou non, un oligo ou polycondensat, cela correspond à:

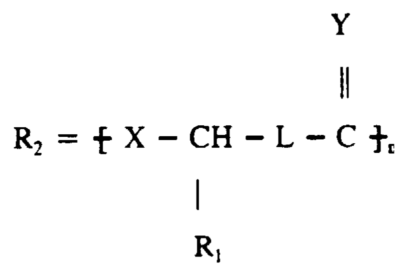

avec n = 1 à 1 000. Pour les dimères et les oligo et poly condensats cyclisés, le :

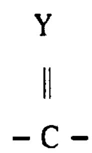

du nième monomère est relié au X du premier monomère.

Les substrats de départ de nature di, oligo et polymérique, peuvent en fait être considérés comme des précurseurs des réels substrats de départ de la réaction de réduction (a), que sont les monomères.

En particulier, mais de façon non limitative, la présente invention vise les esters d'acides α-hydroxycarboxyliques, les énantiomères D ou L d'un ester d'acide lactique étant plus particulièrement préférés.

Les énantiomères de haute pureté optique visés par l'invention sont, bien entendu, directement déductibles des composés **(Id)** cités ci-dessus. On se contentera simplement de rappeler que, parmi les composés chiraux **(If)** préférés, figurent les diols-1,2 et, en particulier, le propane-1,2 diol, ainsi que le butane-1,2 diol.

Il est à considérer que les étapes **a), b), c)** caractéristiques du procédé selon l'invention peuvent s'accommoder de quelques variantes quant à la chronologie de leur mise en oeuvre. De façon préférée, elles sont successives.

Le coréactif est l'un des éléments essentiels du procédé selon l'invention. En respectant les spécifications relatives à la présence d'au moins un hydroxyle et/ou d'un thiol parmi ses substituants, ainsi qu'à sa température d'ébullition à $P_{atm}$, qui est avantageusement supérieure à celle de l'énantiomère **(If)**, ce coréactif est choisi parmi les thiols, tels que l'acide thiosalicylique et/ou les 2 ou 4-chlorobenzylmercaptans et/ou parmi les alcools, tels que les alcools aromatiques et/ou les alcools aliphatiques et/ou les alcools alicycliques et/ou les polyols, notamment ceux de nature glucidique et/ou les acides carboxyliques comprenant au moins un groupement hydroxyle, de préférence parmi les alcools aromatiques et/ou les acides carboxyliques hydroxylés ayant de 4 à 10 carbones.

Plus précisément, le coréactif comprend, avantageusement, deux radicaux XH et/ou YH, de préférence distants de 2 à 4 atomes par le plus court chemin.

Les diphénols, substitutés ou non, et en particulier le pyrocatéchol, le résorcinol et l'hydroquinone forment une classe de coréactifs préférés.

Cette préférence trouve, en partie, son origine dans le fait que de tels composés sont susceptibles de former un cycle avec le produit de transformation de l'hydrure, qui bloque l'obtention de produits **If**, en formant un intermédiaire à l'issue de la réduction **(a)**. En effet, sans vouloir être lié par la théorie, il semble que la cyclisation constitue un mécanisme qui favorise la libération des produits **If**.

Dans une autre classe de coréactifs avantageux, on trouve : l'alcool benzylique, le 1-octanol, le 1-dodécanol, le glycérol, l'acide glycolique, l'acide tartrique et l'acide mandélique.

S'agissant du milieu solvant organique anhydre, il est souhaitable qu'il soit constitué de solvants polaires conformément à une première variante de mise en oeuvre de l'invention et, en particulier, que le ou les constituants dudit milieu soient choisis parmi les glymes ou leurs précurseurs, tels que les polyéthylène-glycol.

Selon une seconde variante de mise en oeuvre relative à la nature du milieu solvant organique anhydre, on sélectionne le ou les constituants dudit milieu parmi les solvants apolaires, de préférence parmi les alkyles, éventuellement hydroxylés et/ou les aryles substitués ou non, notamment par des alkyles et/ou des halogènes ; les carbures benzéniques, tels que le xylène, étant particulièrement préférés.

Pour parfaire l'étape **c** de distillation directe du milieu réactionnel, de façon à séparer l'énantiomère **(If)**, la Demanderesse a eu le mérite de prévoir une disposition avantageuse selon laquelle l'un au moins des constituants du milieu solvant organique anhydre est de nature telle qu'il forme un azéotrope avec le susdit énantiomère **(If)**. En effet, dès lors que ce dernier est déplacé de sa liaison avec le produit de transformation de l'hydrure grâce à l'intervention du coréactif, il peut se mélanger au constituant du solvant considéré et être ainsi récupérable plus aisément et plus rapidement, puisque la température d'ébullition du mélange à la pression atmosphérique normale est inférieure à celle de **(If)** et, a fortiori, à celle du coréactif et des autres résidus du milieu réactionnel.

Une telle disposition procure, en outre, l'avantage de réduire, de façon notable, le coût de revient du procédé.

L'hydrure, utile comme réducteur, est choisi parmi les hydrures de métaux, en tenant compte de considérations techniques et économiques. Les susdits métaux sont, de préférence, des métaux de la colonne IIIA de la classification périodique, sachant que le bore est particulièrement préféré et, plus préférentiellement encore, l'hydrure sélectionné est le $NaBH_4$.

Selon une caractéristique avantageuse, l'étape **c)** de distillation directe est effectuée sous vide, dans un souci d'optimisation.

Sans que cela ne soit limitatif, pour ce qui concerne les substrats et les produits finaux visés, on peut signaler que dans les formules **(If)** et **(Id)**, avantageusement, X et Y représentent l'oxygène, L une liaison simple $\sigma$, $R_1$ est un alkyle de $C_1$ à $C_{10}$, de préférence un méthyle, éthyle ou butyle, $R_2$ est un alcoxyle de $C_1$ à $C_{10}$, de préférence le méthoxyle, l'éthoxyle, butoxyle, l'isobutoxyle.

En fait, **(Id)** est, par exemple, un lactate d'alkyle, de préférence de méthyle et **(If)** l'un des énantiomères du propane-1,2 diol.

Concernant la méthodologie du procédé selon l'invention, la stoechiométrie des réactions est, avantageusement, telle que l'hydrure et le coréactif soient en excès par rapport au substrat **(If)**.

Ainsi, le ratio en normalité **(If)** : hydrure varie, de préférence, de 1 : 1 à 1 : 2.

Concernant le coréactif, ce rapport de normalité varie, de préférence, de 1 : 2 à 1 : 3.

Le procédé suivant l'invention donne accès à des énantiomères de grande pureté optique (ee $\geq$ 95 %) et à faible prix de revient par rapport à ceux obtenus antérieurement. Jusqu'alors limités pour des raisons économiques, les débouchés et les valorisations de tels énantiomères purs devraient se multiplier, en particulier dans le domaine de la synthèse stéréosélective en tant qu'intermédiaires de synthèse ou bien encore dans les domaines pharmaceutiques et agrochimiques, en tant que principes actifs et, enfin, comme précurseur dans la préparation de polymères spéciaux du type cristaux liquides.

Les variantes du procédé selon l'invention, dont notamment celles relatives au mode opératoire, ainsi que tous les nombreux avantages du susdit procédé ressortiront bien des exemples de mise en oeuvre qui suivent, ainsi que des résultats obtenus. notamment sur le plan des puretés et des rendements optiques atteints.

**EXEMPLES**

**EXEMPLE 1: SYNTHÈSE DE R (-) PROPANEDIOL 1,2 À PARTIR DE R (+) D LACTATE DE MÉTHYLE.**

**1.1 SCHÉMA RÉACTIONNEL :**

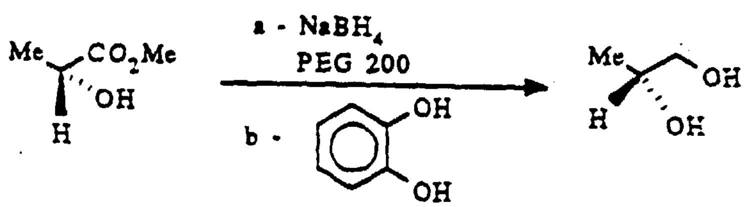


R-(+)-D- Lactate de méthyle

**1.2 MATIÈRES PREMIÈRES :**

| QUANTITES | MOLES | NATURE - PROVENANCE - SPECIFICATIONS |
|---|---|---|
| 100 g | 0,96 (1 eq) | R (+) D lactate de méthyle<br>RHONE POULENC - Usine de MELLE - D = 97,48 % |
| 47 g | 1,25 (1,3 eq) | $NaBH_4$ (Aldrich lot 56524-32) (98 %) |
| 500 ml | | Polyéthylène glycol 200 (Aldrich lot 56524-32) |
| 275 g | 2,50 (2,6 eq) | Pyrocatéchine (Aldrich lot 24152) (98 %) |

**1.3 EQUIPEMENT UTILISÉ :**

**1.3.1** RÉACTION :

Ballon fond rond de 2 1, muni d'une ampoule à addition de solide (vis sans fin), d'un réfrigérant, d'un thermomètre et d'un agitateur mécanique. Une prise d'argon sur le réfrigérant.

**1.3.2** DISTILLATION :

Le réfrigérant est remplacé par une colonne Vigreux de 20 cm + thermomètre + condenseur + collecteur + prise de vide.

**1.4 MODE OPÉRATOIRE :**

| | | |
|---|---|---|
| 0 h | Le lactate de méthyle est dissous dans le polyéthylène glycol 200 sous argon. Le borohydrure est additionné lentement (pendant 8 heures) en agitant et sous argon, de façon à maintenir la température entre 40 et 60° C et à limiter les mousses dues au dégagement d'hydrogène (après 2 h 25 mn, l'addition est interrompue pendant 1 h, au cours de laquelle la température est spontanément redescendue à 28° C). Après la fin de l'addition, le mélange réactionnel est agité une nuit à température ambiante ($\theta$ = 28° C). | |
| 24 h | La pyrocatéchine est additionnée en plusieurs portions (sur 1 h) au milieu réactionnel blanc et épais, puis le tout est chauffé à 100° C pendant 2 h. Le milieu est refroidi à 30° C, le réfrigérant est remplacé par une colonne Vigreux et un condenseur et l'ensemble est mis sous vide, puis chauffé. Six fractions ont été collectée (le méthanol n'a pas été collecté). | |
| 22 mbar | 85° C<br>↓<br>95° C | 1 = 2,54 g<br>2 = 8,70 g<br>3 = 7,72 g<br>4 = 11,06 g |
| 28 mbar | 104-95° C | 5 = 15,97 g |
| 28 mbar | 90-97° C | 6 = 19,27 g ($\theta_{masse}$ = 160° C)<br>Total des fractions = 65,26 g.<br>Rendement = 89,4 %. |

**1.5 RÉSULTATS:**

Les analyses quantitatives et qualitatives des fractions obtenues par distillation sont effectuées par chromatographie en phase gazeuse sur un chromatographe de type VARIAN 6000. La dérivatisation est du type O.TFA.
La pureté énantiomérique du R (-) propanediol-1,2 est de 97,16 %, alors que celle du R (+) lactate de méthyle utilisé était de 97,48 %. Le rendement optique est donc de 99,7 %.

**EXEMPLE 2: SYNTHÈSE DE S (+) PROPANEDIOL 1,2 À PARTIR DE S (-) LACTATE DE MÉTHYLE.**

Cet exemple 2 est semblable à l'exemple 1, aux différences près que :

- le produit de départ (Id) est le S (-) lactate de méthyle (Aldrich 98 %, lot 82604-82),
- le solvant est le polyéthylène glycol 300 (Aldrich lot B 57339),
- les quantités stoechiométriques ont été divisées par 10 par rapport à l'exemple 1. Après distillation, on recueille trois fractions A, B, C, essentiellement constituées de propane 1,2 diol, la fraction C étant toutefois légèrement plus riche que les fractions A et B en pyrocatéchine. Les analyses en résonance magnétique nucléaire confirment la structure des produits obtenus.

La chromatographie en phase gazeuse permet d'établir que le rendement chimique en propanediol est de 99 %, tandis que le rendement optique en énantiomère S (+) propanediol-1,2 est de 100 %.

**EXEMPLE 3 : SYNTHÈSE DE S (+) PROPANEDIOL À PARTIR DE S (-) LACTATE DE MÉTHYLE.**

L'exemple 3 est identique à l'exemple 2, aux différences près que :

- l'acide tartrique remplace la pyrocatéchine à titre de coréactif,
- on incorpore du xylène dans le milieu réactionnel préalablement à l'étape **c)** de distillation directe,
- on met en oeuvre une étape préalable d'évaporation avant réduction (96° C, 16 millibars pendant 20 mn), de manière à éliminer les sous-produits de faible poids moléculaire indésirables contenus dans le polyéthylène glycol.

Lors de la distillation on recueille, tout d'abord, un premier azéotrope méthanol/p-xylène, le méthanol étant un sous-produit de la réduction par les hydrures, puis, dans un second temps, un deuxième azéotrope propanediol/o-xylène, respectivement à des températures de 64 et 138° C.
Le propanediol et le xylène ne sont pas miscibles entre eux, donc se séparent très aisément.
Le rendement chimique en propanediol est de 78 % environ, tandis que le rendement optique s'établit autour de 98 %.

**EXEMPLE 4 : SYNTHÈSE DE S (+) PROPANEDIOL 1,2 À PARTIR DE S (-) LACTATE DE MÉTHYLE.**

Dans cet exemple, la méthodologie générale, donnée dans les exemples 1 à 3, est reprise. Le présent exemple 4 correspond sensiblement à l'exemple 2, à quelques modifications près, pour ce qui concerne les réactifs et le mode opératoire.

**4.1 RÉACTIFS :**

Le polyéthylène glycol 300 est remplacé par du xylène, du type de celui commercialisé par la Société PROLABO sous la dénomination "NORMAPUR" (mélange d'isomères).

**4.2 MODE OPÉRATOIRE :**

| | |
|---|---|
| 0 h | $\theta$ = 22° C. On charge 500 ml de xylène, puis le borohydrure de sodium en pied pour former un mélange hétérogène. Agitation à 197 t/min. |
| 10 min | $\theta$ = 22° C. On refroidit par un bain d'eau, coulée du lactate de méthyle en 46 min. |
| 55 min | $\theta$ = 47° C. Exothermie et dégagement gazeux important. |
| | $\theta$ = 55° C. Chauffage pendant 5 h 10 min. |
| 6 h 05 | $\theta$ = 58° C. Refroidissement. |

(suite)

| | |
|---|---|
| 6 h 35 | θ = 38° C. Coulée de la solution de P. C. (dans du méthanol) pendant 53 min sur le mélange réactionnel (agitation à 437 t/min).<br>Augmentation de volume avec formation de beaucoup de mousse. |
| 7 h | θ = 48° C. Apparition d'un précipité blanc.<br>La masse réactionnelle épaissit beaucoup. 100 ml de MeOH sont ajoutés. |
| 7 h 45 | La coulée est finie. Chauffage et distillation de :<br>(i) méthanol/p-xylène aséotrope (θ = 64° C),<br>(ii) propanediol/o-xylène azéotrope (θ = 134° C).<br>Ajout de 200 ml de xylène.<br>Le propanediol brut est isolé par décantation du distillat = 65,54 g.<br>Ce brut est distillé :<br>- azéotrope o-xylène/propanediol (θ = 134° C),<br>- propanediol pur (sous 25 mbars) = 53,70 g (θ = 87° C). |

**4.3 RÉSULTATS ET ANALYSES :**

La RMN confirme la structure des produits obtenus et la CPG permet de calculer, pour la deuxième fraction, un rendement chimique en propanediol de 100 % pur et un rendement optique de 100 % pur de S (+) propanediol.

Cet exemple avec le xylène est particulièrement intéressant, puisqu'il permet d'obtenir des énantiomères de pureté élevée en mettant en oeuvre des températures de distillation plus basses que celles employées dans les autres exemples ci-avant.

**Revendications**

1. Procédé de préparation de composés de haute pureté énantiomérique de formule :

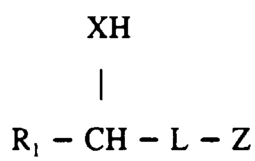

**(If)**

dans laquelle :

- 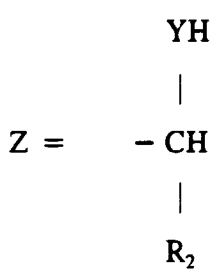
- $R_1$, $R_2$ sont identiques ou différents et représentent l'hydrogène ou un radical hydrocarboné aliphatique et/ou alicyclique et/ou aromatique et/ou hétérocyclique, de préférence un hydrogène ou un alkyle,
- X et Y sont identiques ou différents et représentent l'oxygène ou le soufre,
- et L représente une simple liaison covalente σ ou un groupement alkyle à 1 ou 2 C,

à partir de composés chiraux purs répondant à la formule **(Id)** qui correspond à la formule **(If)** dans laquelle :

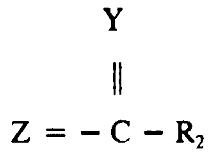

avec $R_2$ et Y tels que définis ci-dessus, $R_2$ pouvant, en outre, représenter un radical $NH_2$, alcoxyle, S-alkylé, ou :

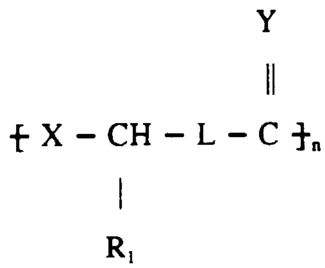

avec n = 1 à 1 000,
procédé du type de ceux faisant intervenir une réduction de composé **(Id)** à l'aide d'au moins un réducteur du type hydrure,

caractérisé en ce que, en milieu anhydre :

**a)** - on effectue la réduction du composé **(Id)** en composé **(If)** dans un milieu solvant organique,
**b)** - on ajoute au milieu réactionnel au moins un coréactif:

- substitué par au moins un radical XH ou YH, de préférence au moins deux,
- apte à déplacer le composé **(If)** dans l'intermédiaire issu de l'étape a précédente,
- et possédant une température d'ébullition à $P_{atm}$ normale supérieure à celle du composé **(If)**,

**c)** - on distille directement le milieu réactionnel de manière à récupérer ce composé **(If)**.

**2.** Procédé selon la revendication 1, caractérisé en ce que les étapes **a)**, **b)**, **c)** sont successives.

**3.** Procédé selon la revendication 1 ou la revendication 2, caractérisé en ce que l'on choisit le coréactif parmi les thiols, tels que l'acide thiosalicylique et/ou les 2 ou 4-chlorobenzylmercaptans, et/ou parmi les alcools, tels que les alcools aromatiques et/ou les alcools aliphatiques et/ou les alcools alicycliques et/ou les polyols, notamment ceux de nature glucidique, et/ou les acides carboxyliques comprenant au moins un groupement hydroxyle, de préférence parmi les alcools aromatiques et/ou les acides carboxyliques hydroxylés ayant de 4 à 10 carbones.

**4.** Procédé selon la revendication 3, caractérisé en ce que le coréactif comprend deux radicaux XH et/ou YH, de préférence distants de 2 à 4 atomes par le plus court chemin.

**5.** Procédé selon la revendication 4, caractérisé en ce que le coréactif est choisi parmi les diphénols substitués ou non et, de préférence, parmi les composés suivants :

pyrocatéchol - résorcinol - hydroquinone

**6.** Procédé selon la revendication 3, caractérisé en ce que le coréactif est sélectionné parmi la liste suivante de composés :

alcool benzylique-1-octanol-1-dodécanol- glycérol-acide glycolique - acide tartrique - acide mandélique

**7.** Procédé selon l'une quelconque des revendications 1 à 6, caractérisé en ce que l'on sélectionne le (ou les) constituant(s) du milieu solvant organique anhydre parmi les solvants polaires, de préférence parmi les glymes ou leurs précurseurs, tels que les polyéthylène glycols.

**8.** Procédé selon l'une quelconque des revendications 1 à 6, caractérisé en ce que l'on sélectionne le (ou les) constituant(s) du milieu solvant organique anhydre parmi les solvants apolaires, de préférence parmi les alkyles, éventuellement hydroxylés et/ou les aryles substitués ou non, notamment par des alkyles et/ou des halogènes ; les carbures benzéniques, tels que le xylène, étant particulièrement préférés.

**9.** Procédé selon l'une quelconque des revendications 1 à 8, caractérisé en ce que l'un au moins des constituants du milieu solvant est choisi de telle sorte qu'il forme un azéotrope avec le composé **(If)**, une fois celui-ci déplacé dans l'intermédiaire issu de l'étape **a**, par le coréactif.

**10.** Procédé selon l'une quelconque des revendications 1 à 9, caractérisé en ce que l'on met en oeuvre un hydrure choisi parmi les hydrures de métaux, de préférence de métaux de la colonne III A de la classification périodique, le bore étant particulièrement préféré et, plus préférentiellement encore, on sélectionne le $NaBH_4$.

**11.** Procédé selon l'une quelconque des revendications 1 à 10, caractérisé en ce que l'étape c s'opère sous vide.

**12.** Procédé selon l'une quelconque des revendications 1 à 11, caractérisé en ce que, dans les formules **(If)** et **(Id)** :

- X, Y représentent l'oxygène,
- L une liaison simple σ,
- $R_1$ est un alkyle de $C_1$ à $C_{10}$, de préférence un méthyle, éthyle ou butyle,
- $R_2$ est un alcoxyle de $C_1$ à $C_{10}$, de préférence un méthoxyle, éthoxyle, butoxyle, isobutoxyle.

**13.** Procédé selon l'une quelconque des revendications 1 à 12, caractérisé en ce que :

- **(Id)** = lactate d'alkyle, de préférence de méthyle,
- **(If)** = propane 1,2 diol.

**Patentansprüche**

**1.** Verfahren zur Herstellung von Verbindungen mit hoher Enantiomerenreinheit der Formel:

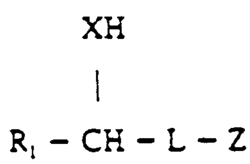

(If)

in der:

- Z =

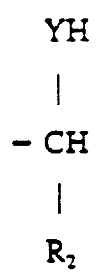

- $R_1$, $R_2$ gleich oder verschieden sind und Wasserstoff oder einen aliphatischen und/oder alicyclischen und/oder aromatischen und/oder heterocyclischen Kohlenwasserstoffrest vorzugsweise ein Wasserstoffatom oder einen Alkylrest darstellen,
- X und Y gleich oder verschieden sind und Sauerstoff oder Schwefel darstellen,
- und L eine kovalente σ-Einfachbindung oder eine Alkylgruppe mit 1 oder 2 C darstellt,

aus reinen chiralen Verbindungen, die der Formel (Id) gleichkommen, die der Formel (If) entspricht, in der:

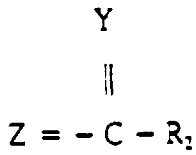

mit $R_2$ und Y wie oben definiert, wobei $R_2$ außerdem einen $NH_2$-, Alkoxy-, S-Alkyl-Rest oder:

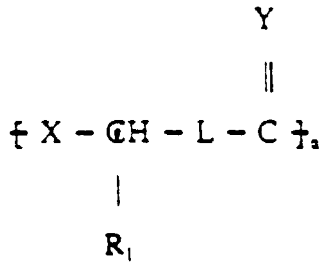

mit n = 1 bis 1 000, darstellen kann,
ein Verfahren vom Typ derjenigen, bei denen eine Reduktion der Verbindung (Id) mit Hilfe wenigstens eines Reduktionsmittels vom Hydrid-Typ beteiligt ist.

dadurch gekennzeichnet, daß in wasserfreiem Medium:

a) - man die Reduktion der Verbindung (Id) zur Verbindung (If) in einem organischen Lösungsmittelmedium ausführt,
b) - man dem Reaktionsmedium wenigstens ein Co-Reagens hinzufügt:

- das mit wenigstens einem XH- oder YH-Rest, vorzugsweise mit wenigstens zwei, substituiert ist,
- das fähig ist, die Verbindung (If) in dem aus dem vorhergehenden Schritt a hervorgegangenen Zwischenprodukt zu ersetzen,
- und das eine Siedetemperatur bei Normalluftdruck $P_{atm}$ besitzt, die höher als die der Verbindung (If) ist,

c) - man das Reaktionsmedium direkt destilliert, um diese Verbindung (If) zu gewinnen.

**2.** Verfahren gemäß Anspruch 1, dadurch gekennzeichnet, daß die Schritte a), b) und c) aufeinander folgen.

**3.** Verfahren gemäß Anspruch 1 oder Anspruch 2, dadurch gekennzeichnet, daß man das Co-Reagens unter den Thiolen, wie Thiosalicylsäure und/oder 2- oder 4-Chlorbenzylmercaptan, und/oder den Alkoholen, wie den aromatischen Alkoholen und/oder den aliphatischen Alkoholen und/oder den alicyclischen Alkoholen und/oder den Polyolen, insbesondere den zuckerartigen, und/oder den Carbonsäuren mit wenigstens einer Hydroxylgruppe, vorzugsweise unter den aromatischen Alkoholen und/oder den Hydroxycarbonsäuren mit 4 bis 10 Kohlenstoffatomen auswählt.

**4.** Verfahren gemäß Anspruch 3, dadurch gekennzeichnet, daß das Co-Reagens zwei XH- und/oder YH-Reste umfaßt, die auf dem kürzesten Weg vorzugsweise 2 bis 4 Kohlenstoffatome entfernt sind.

**5.** Verfahren gemäß Anspruch 4, dadurch gekennzeichnet, daß das Co-Reagens unter den substituierten oder nicht substituierten Diphenolen und vorzugsweise unter den folgenden Verbindungen ausgewählt ist:
Brenzcatechin - Resorcin - Hydrochinon

**6.** Verfahren gemäß Anspruch 3, dadurch gekennzeichnet, daß das Co-Reagens aus der folgenden Liste von Verbindungen ausgewählt ist:
Benzylalkohol - 1-Octanol - 1-Dodecanol - Glycerin - Glykolsäure - Weinsäure - Mandelsäure

**7.** Verfahren gemäß einem der Ansprüche 1 bis 6, dadurch gekennzeichnet, daß man den (oder die) Bestandteil(e)

des wasserfreien organischen Lösungsmittelmediums unter den polaren Lösungsmitteln, vorzugsweise unter den Glykolethem oder deren Vorstufen, wie den Polyethylenglykolen, auswählt.

**8.** Verfahren gemäß einem der Ansprüche 1 bis 6, dadurch gekennzeichnet, daß man den (oder die) Bestandteil(e) des wasserfreien organischen Lösungsmittelmediums unter den unpolaren Lösungsmitteln, vorzugsweise unter den gegebenenfalls hydroxylierten Alkylen und/oder den insbesondere durch Alkyle und/oder Halogenide substituierten oder nicht substituierten Arylen auswählt; die Benzol-Kohlenwasserstoffe, wie Xylol, sind besonders bevorzugt.

**9.** Verfahren gemäß einem der Ansprüche 1 bis 8, dadurch gekennzeichnet, daß wenigstens einer der Bestandteile des Lösungsmittelmediums so gewählt ist, daß er mit der Verbindung (If), wenn diese in dem aus dem Schritt a hervorgegangenen Zwischenprodukt einmal durch das Co-Reagens ersetzt ist, ein Azeotrop bildet.

**10.** Verfahren gemäß einem der Ansprüche 1 bis 9, dadurch gekennzeichnet, daß man ein Hydrid einsetzt, das unter den Hydriden der Metalle, vorzugsweise der Metalle der Gruppe IIIA des Periodensystems ausgewählt ist, wobei Bor ganz besonders bevorzugt ist, und man noch stärker bevorzugt $NaBH_4$ auswählt.

**11.** Verfahren gemäß einem der Ansprüche 1 bis 10, dadurch gekennzeichnet, daß sich der Schritt c unter Vakuum vollzieht.

**12.** Verfahren gemäß einem der Ansprüche 1 bis 11, dadurch gekennzeichnet, daß in den Formeln (If) und (Id):

- X, Y Sauerstoff darstellen,
- L eine $\sigma$-Einfachbindung darstellt,
- $R_1$ ein $C_1$- bis $C_{10}$-Alkylrest, vorzugsweise ein Methyl-, Ethyl- oder Butylrest ist,
- $R_2$ ein $C_1$- bis $C_{10}$-Alkoxyrest, vorzugsweise ein Methoxy-, Ethoxy-, Butoxy-, Isobutoxyrest ist.

**13.** Verfahren gemäß einem der Ansprüche 1 bis 12, dadurch gekennzeichnet, daß:

- (Id) = Alkyl-, vorzugsweise Methyllactat,
- (If) = Propan-1,2-diol.

**Claims**

**1.** Process for the preparation of compounds of high enantiomeric purity, of formula:

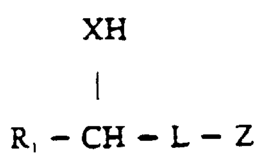

(If)

in which:

- Z = 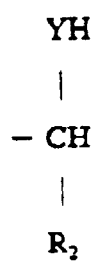

- $R_1$ and $R_2$ are identical or different and denote hydrogen or an aliphatic and/or alicyclic and/or aromatic and/or heterocyclic hydrocarbon radical, preferably a hydrogen or an alkyl,

- X and Y are identical or different and denote oxygen or sulphur, and
- L denotes a σ covalent single bond or a $C_1$ or $C_2$ alkyl group,

from pure chiral compounds corresponding to the formula (Id), which corresponds to the formula (If) in which:

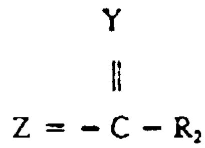

with $R_2$ and Y as defined above, it being additionally possible for $R_2$ to denote an $NH_2$, alkoxy, or alkylated S radical or:

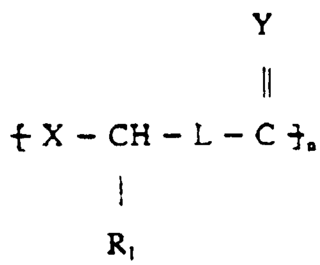

with n = 1 to 1000,

of the type of those involving a reduction of a compound (Id) with the aid of at least one reducing agent of the hydride type,

characterized in that, in an anhydrous mixture:

a) - the reduction of the compound (Id) to a compound (If) is performed in an organic solvent medium,

b) - there is added to the reaction mixture at least one coreactant which is:

- substituted with at least one radical XH or YH, preferably at least two,
- capable of displacing the compound (If) in the intermediate originating from the preceding stage a, and
- possessing a boiling temperature at normal $P_{atm}$ which is higher than that of the compound (If),

c) - the reaction mixture is distilled directly so as to recover this compound (If).

**2.** Process according to claim 1, characterized in that the stages a), b) and c) are successive.

**3.** Process according to claim 1 or claim 2, characterized in that the coreactant is chosen from thiols such as thiosalicylic acid and/or 2- or 4-chlorobenzyl mercaptans and/or from alcohols such as aromatic alcohols and/or aliphatic alcohols and/or alicyclic alcohols and/or polyols, especially those of glucidic nature, and/or carboxylic acids containing at least one hydroxyl group, preferably from aromatic alcohols and/or hydroxylated carboxylic acids containing from 4 to 10 carbons.

**4.** Process according to claim 3, characterized in that the coreactant contains two radicals XH and/or YH, preferably from 2 to 4 atoms apart via the shortest route.

**5.** Process according to claim 4, characterized in that the coreactant is chosen from substituted or unsubstituted diphenols and preferably from the following compounds:

pyrocatechol - resorcinol - hydroquinone.

**6.** Process according to claim 3, characterized in that the coreactant is selected from the following list of compounds:

benzyl alcohol - 1-octanol - 1-dodecanol - glycerol - glycolic acid - tartaric acid - mandelic acid.

**7.** Process according to any one of claims 1 to 6, characterized in that the constituent(s) of the anhydrous organic solvent medium is (are) chosen from polar solvents, preferably from glymes or their precursors, such as polyethylene glycols.

**8.** Process according to any one of claims 1 to 6, characterized in that the constituent(s) of the anhydrous organic solvent medium is (are) selected from apolar solvents, preferably from optionally hydroxylated alkyls and/or aryls which are unsubstituted or substituted, especially with alkyls and/or halogens, benzene-related hydrocarbons such as xylene being particularly preferred.

**9.** Process according to any one of claims 1 to 8, characterized in that at least one of the constituents of the solvent medium is chosen so that it forms an azeotrope with the compound (If), once the latter is displaced by the coreactant in the intermediate originating from stage a.

**10.** Process according to any one of claims 1 to 9, characterized in that a hydride chosen from metal hydrides, preferably of metals of column IIIA of the Periodic Classification, boron being particularly preferred, is used and, still more preferably, $NaBH_4$ is selected.

**11.** Process according to any one of claims 1 to 10, characterized in that the stage c is performed under vacuum.

**12.** Process according to any one of claims 1 to 11, characterized in that, in formulae (If) and (Id) :

- X and Y denote oxygen,
- L a σ single bond,
- $R_1$ is a $C_1$-$C_{10}$ alkyl, preferably a methyl, ethyl or butyl,
- $R_2$ is a $C_1$-$C_{10}$ alkoxy, preferably methoxy, ethoxy, butoxy or isobutoxy.

**13.** Process according to any one of claims 1 to 12, characterized in that:

- (Id) = alkyl, preferably methyl, lactate,
- (If) =1,2-propanediol.